(19) Europäisches Patentamt
European Patent Office
Office européen des brevets

(11) EP 2 838 502 B1

# (12) EUROPEAN PATENT SPECIFICATION

(45) Date of publication and mention of the grant of the patent:
**14.02.2018 Bulletin 2018/07**

(21) Application number: **13716275.6**

(22) Date of filing: **11.04.2013**

(51) Int Cl.:
*A61K 8/58* (2006.01)
*A61Q 17/04* (2006.01)
*A61Q 19/00* (2006.01)
*A61Q 5/00* (2006.01)
*A61K 31/38* (2006.01)
*C07F 7/18* (2006.01)
*A61K 31/366* (2006.01)
*A61Q 5/06* (2006.01)

(86) International application number:
**PCT/EP2013/057584**

(87) International publication number:
**WO 2013/156387 (24.10.2013 Gazette 2013/43)**

## (54) WATER RESISTANT COMPOSITIONS CONTAINING A HETEROCYCLIC COMPOUND AND AN ALKOXYSILANE

WASSERRESISTENTE ZUSAMMENSETZUNGEN DIE EINE HETEROCYKLISCHE VERBINDUNG UND EIN ALKOXYSILAN ENTHALTEN

COMPOSITIONS RÉSISTANT À L’EAU CONTENANT UN COMPOSÉ HÉTÉROCYCLIQUE ET UN ALCOXYSILANE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**17.04.2012 US 201213448440**
**17.04.2012 US 201261625105 P**
**18.03.2013 US 201313845600**

(43) Date of publication of application:
**25.02.2015 Bulletin 2015/09**

(73) Proprietor: **L’Oréal**
**75008 Paris (FR)**

(72) Inventors:
- **NGUYEN, Nghi Van**
  **Edison, New Jersey 08820 (US)**
- **SHMUYLOVICH, Gregory**
  **Springfield, New Jersey 07081 (US)**
- **CHIOU, Catherine**
  **Saddle Brook, New Jersey 07663 (US)**

(74) Representative: **Casalonga**
**Casalonga & Partners**
**Bayerstraße 71/73**
**80335 München (DE)**

(56) References cited:
**WO-A1-2009/061360**
**US-A- 3 419 517**
**US-A- 4 104 296**
**US-A- 4 591 652**
**US-A1- 2010 255 046**
**US-A1- 2010 310 491**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 838 502 B1

## Description

### FIELD OF THE INVENTION

The present invention relates to water resistant compositions and methods of using these compositions on various substrates. More particularly, the invention is directed to a method of imparting water resistance onto a keratinous substrate comprising a composition containing (a) a reaction product of at least one heterocyclic compound chosen from lactone compounds and their thiocarbonyl analogs, thiolactone compounds and their thiocarbonyl analogs, and lactam compounds and their thiocarbonyl analogs with at least one alkoxysilane having at least one amino substituent, and (b) optionally, at least one carrier.

This invention is further directed to a specific cosmetic composition useful for implementing said method.

### BACKGROUND OF THE INVENTION

Consumer products such as cosmetics, personal care and household products are designed to provide a wide range of desirable properties and benefits to various substrates such as keratinous substrates, hard surfaces, and other non-keratinous substrates, for example, fabrics and personal articles. Generally, these products deliver benefit agents to a substrate. These products can also be designed to protect said substrates from extreme environmental conditions or from physical contact such as rubbing or from exposure to water, humidity, moisture or other liquids.

In particular, when keratinous substrates such as skin and hair are exposed to environmental conditions, for example, high or low humidity or to ultraviolet radiation from the sun, these substrates can lose many of their desirable properties and even become damaged. The appearance and condition of skin and hair can also be affected by ultraviolet exposure and aging. For example, skin can become dry and flaky, while hair can dry out and lose its shine or color or become frizzy and less manageable under low and high humidity conditions. Under low humidity conditions, hair can dry out and dried-out hair tends to be less shiny and more brittle. Conversely, under high humidity conditions, hair tends to absorb water causing hair to lose its shape and become unmanageable and unattractive. Furthermore, hair can lose its desirable attributes due to physical stress on the hair such as brushing and application of heat.

As a result, consumers continue to seek products such as sunscreens, skin care, hair care and hair cosmetic compositions which protect and enhance the appearance of skin and hair as well as reduce the deleterious effects of adverse environmental conditions, photo-damage, and physical stress. It is thus important to ensure that the beneficial properties of such products remain on the skin and hair by making these products water resistant and/or able to provide a protective barrier or hydrophobicity to the skin and hair as well as durable or long-lasting hydrophobicity.

Water resistant products do not easily "run off" or wash off when the skin and hair are exposed to water, rain, and tears or upon sweating nor easily transfer from the skin or hair because of normal every day activity. These products also tend to have long wearing and transfer-resistant properties, that is, they adhere longer to surfaces and to keratinous substrates. Commercial products which have these properties may require high amounts of paraffin, fatty alcohols, petrolatum, Vaseline, waxes or oils, e.g., mineral oil. Other customary barrier agents are silicones and conventional film forming agents or polymers. However, such ingredients still present many disadvantages; for example, high levels of oils and hydrocarbon-based ingredients make the skin or hair greasy, waxes can give an unpleasant aesthetic look and feel, and silicones can leave residues and may give an uncomfortable feel and wear.

Thus, there still exists a need to find other materials or compositions that can provide a water-resistant and/or protective barrier to the skin and hair and which do not require the customary barrier agents.

Thus, it is an object of the present invention to provide materials and compositions which provide a water resistant protective barrier onto skin and hair, as well as impart durable or long lasting hydrophobicity and improve the water resistance of cosmetic and personal care compositions. It is also an object of the present invention to provide a water resistant composition that can function as a carrier and/or matrix for desired benefit agents used to benefit skin and hair.

### BRIEF SUMMARY OF THE INVENTION

The present invention relates to a composition containing:

(a) a reaction product of:

(i) at least one heterocyclic compound chosen from $\gamma$-thiobutyrolactone, meadowfoam delta-lactone and mixtures thereof; and

(ii) 3-aminopropyltriethoxysilane; and

(b) optionally, at least one carrier; and

(c) at least one benefit agent chosen from sunscreen agents, cosmetically active agents, dermatologically active agents, humectants, moisturizing agents, colorants, hair straightening/relaxing agents, film forming agents, shine agents, conditioning agents, reducing agents, emollients, vitamins, antidandruff agents, plant extracts, antiopersirants, and pharmaceutical agents;

wherein the composition is water resistant.

Furthermore, the present invention relates to a method of imparting water resistance onto a substrate, the method comprising applying onto the substrate, a composition containing:

(a) a reaction product of:

(i) at least one heterocyclic compound chosen from lactone compounds and their thiocarbonyl analogs, thiolactone compounds and their thiocarbonyl analogs, and lactam compounds and their thiocarbonyl analogs; and

(ii) at least one alkoxysilane having at least one amino substituent; and

(b) optionally, at least one carrier;

DETAILED DESCRIPTION OF THE INVENTION

As used herein, the expression "at least one" means one or more and thus includes individual components as well as mixtures/combinations.

Other than in the operating examples, or where otherwise indicated, all numbers expressing quantities of ingredients and/or reaction conditions are to be understood as being modified in all instances by the term "about," meaning within $\pm$ 10% of the indicated number.

"Keratinous substrates" as used herein, include, but are not limited to skin, hair, lips, and eyelashes.

"Permeating through" refers to the movement of a substance, such as water, into or out of the keratinous substrate. The term may also refer to the loss of water or to the uptake/absorption of water by the keratinous substrate.

"Film former or "film forming agent" as used herein means a polymer or resin that leaves a film on the substrate to which it is applied, for example, after a solvent accompanying the film former has evaporated, absorbed into and/or dissipated on the substrate.

"Substituted" as used herein, means comprising at least one substituent. Non-limiting examples of substituents include atoms, such as oxygen atoms and nitrogen atoms, as well as functional groups, such as hydroxyl groups, ether groups, alkoxy groups, acyloxyalky groups, oxyalkylene groups, polyoxyalkylene groups, carboxylic acid groups, amine or amino groups, acylamino groups, amide groups, halogen containing groups, ester groups, thiol groups, sulphonate groups, thiosulphate groups, siloxane groups, and polysiloxane groups. The substituent(s) may be further substituted.

"Non-reactive solvent" as used herein, refers to a solvent comprising one or more compounds which do not have functional groups that could compete with the reaction of the at least one alkoxysilane having at least one amino substituent with the heterocyclic ring of the heterocyclic compound chosen from lactone compounds and their thiocarbonyl analogs, thiolactone compounds and their thiocarbonyl analogs, and lactam compounds and their thiocarbonyl analogs of the present invention.

The term "anhydrous" as used herein is intended to mean that the composition is either completely free of unbound water or contains substantially no unbound water, such as, for example, no more than 1% by weight, such as no more than 0.5% by weight, based on the weight of each composition.

As used herein, the phrase "salts and derivatives thereof" is intended to mean all salts and derivatives comprising the same functional structure as the compound they are referring to, and that have similar properties.

"Monoacid" as used herein, refers to a compound other than the heterocyclic compound chosen from lactone compounds and their thiocarbonyl analogs, thiolactone compounds and their thiocarbonyl analogs, and lactam compounds and their thiocarbonyl analogs and the alkoxysilane having at least one amino substituent of the present invention, wherein said monoacid compound has a single carboxylic group.

As used herein, the term "applying" a composition to a keratinous substrate with a composition is intended to mean contacting the keratinous substrate, for example skin or hair, with at least one of the compositions of the invention, in any manner.

As used herein, the terms "straightening" or "straighten" or "relaxing" or "relax" the hair mean to remove the curl from the hair or reduce the degree of curl of the hair. It also means changing the shape of hair or the degree of curl in the hair to make the hair more straight. It can also mean removing or reducing the frizziness of the hair.

As used herein, "cosmetically acceptable" means that the item in question is compatible with any human keratinous substrate or material, such as human hair and human skin.

As used herein, "carrier" means a carrier that is compatible with any human keratinous substrate or material, such as human hair and human skin.

As used herein, "conditioning" means imparting to a keratinous substrate such as hair or skin at least one property chosen from combability, manageability, moisture-retentivity, luster, shine, smoothness, and softness. In case of combing, the level of conditioning on the keratinous substrate such as hair is evaluated by measuring, and comparing, the ease of combability of the treated hair and of the untreated hair in terms of combing work (gm-in).

As used herein, "formed from," means obtained from chemical reaction of, wherein "chemical reaction," includes spontaneous chemical reactions and induced chemical reactions. As used herein, the phrase "formed from," is open ended and does not limit the components of the composition to those listed.

It was surprisingly and unexpectedly discovered that the above-disclosed compositions and reaction products are water resistant and can be employed to impart water resistance, as well as durable or long lasting hydrophobicity and/or a protective barrier onto various substrates such as keratinous substrates and non-keratinous surfaces.

The compositions and/or reaction products of the present invention can also function as carriers and/or matrices for desired benefit or additive agents by delivering cosmetic or other desirable properties to various substrates, while allowing these properties to remain longer on the substrates.

The presence of a monoacid is not required in the compositions of the present invention in order for said compositions to be water resistant and to impart properties of product durability and lasting hydrophobicity, as well as a protective barrier, onto a substrate. In addition, the compositions of the present invention do not require the use of a film former chosen from traditional film formers in order to be water-resistant.

The compositions of the present invention may consist of cosmetic compositions for application onto hair and skin and employed to form a water-resistant protective barrier on hair, for example, to help to keep moisture in the hair and allow hair to maintain its shine or to keep moisture out of the hair under high humidity conditions and improve manageability and condition of the hair.

Such cosmetic compositions can also be employed to alter or maintain the shape of hair and could be useful to style hair, straighten hair, curl hair, retain hair curl or retain the style of the hair. These compositions can also be designed to inhibit color fading in both dyed and naturally colored hair and could be useful in coloring or altering the color of hair.

The compositions of the present invention is also useful in cosmetic applications onto substrates such as skin, lips, nails and eyelashes, such as makeup, skin care and suncare products, particularly, in allowing beneficial ingredients in these products to remain longer on these substrates.

The compositions of the present invention are water-resistant such that they are not easily removed from the substrate and/or not easily transferred from the substrate over time by normal everyday activity.

HETEROCYCLIC COMPOUNDS CHOSEN FROM LACTONE COMPOUNDS AND THEIR THIOCARBONYL ANALOGS, THIOLACTONE COMPOUNDS AND THEIR THIOCARBONYL ANALOGS, AND LACTAM COMPOUNDS AND THEIR THIOCARBONYL ANALOGS

The heterocyclic compounds chosen from lactone compounds and their thiocarbonyl analogs used in the method of the present invention include, but are not limited to, cyclic ester compounds comprising a heterocyclic ring wherein the heteroatom on the heterocyclic ring is oxygen and which are represented by the general formula (I):

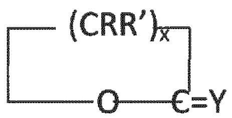

(I)

and wherein:

R or R', independently, is H; a hydrocarbon radical containing from 1 to 40 carbon atoms which may be saturated or unsaturated, linear or branched, substituted or unsubstituted; or a substituent other than H or said hydrocarbon radical, such as a hydroxy group, an amino group, a sulfhydryl group, an aryl group and a halogen;

X represents an integer of 1 or more, such as an integer from between 1 to 10 or such as from 1 to 8 or such as from 1 to 6;

Y is oxygen (0) or sulfur (S) ; and

the heterocyclic ring is saturated or unsaturated.

More particularly, the lactone compounds used in the method of the present invention include, but are not limited to, those lactone compounds comprising 3- to 8-membered rings (including the oxygen on the heterocyclic ring and the carbonyl carbon).

Examples of lactone compounds are α-lactones (3-membered ring alpha-lactones), β-lactones (4-membered

ring beta-lactones), γ-lactones (5-membered ring gamma-lactones), 5-lactones (6-membered ring delta-lactones), and ε-lactones (8-membered ring epsilon-lactones).

In preferred embodiments of the method of the present invention, the lactone compounds of the present invention include, but are not limited to, 5-lactone (delta-lactone) compounds corresponding to the formula (II):

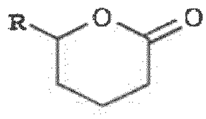

(II)

wherein R is chosen from H, a hydrocarbon radical containing from 1 to 40 carbon atoms, a hydroxyl group, an amino group, a sulfhydryl group, an aryl group, and a halogen;
wherein when R is a hydrocarbon radical, R may be linear or branched, saturated or unsaturated, substituted or unsubstituted;
wherein the carbon atoms on the lactone ring, other than the R-substituted carbon atom and the carbon on the ketone group, may be substituted; and
wherein the heterocyclic ring is saturated or unsaturated.

Examples of δ-lactone compounds used in the method of the present invention are meadowfoam delta-lactone, delta-octalactone, delta-decalactone, delta-nonalactone, undecanoic delta-lactone, delta-dodecalactone, massoia lactone (or 5-pentylpent-2-en-5-olide), jasmine lactone (or Z-2-pentenylpentan-5-olide), 6-Pentyl-alpha-pyrone (or 5-Pentylpenta-2,4-dien-5-olide) delta-valerolactone, galactonolactone, glucono delta-lactone, hexadecanolactone, and mevalonolactone.

Examples of γ-lactone compounds used in the method of the present invention are gamma valerolactone, gamma hexalactone, gamma hepatalactone, gamma octalactone, gamma nonalactone, gamma decalactone, gamma undecalactone and gamma dodecalactone.

The at least one lactone compound used in the method of the present invention as based on formula (I) can be a thiocarbonyl analog of the lactone compound wherein Y in formula (I) is sulfur.

A particularly preferred lactone compound of formula (I) for use in the method of the present invention is the δ-lactone compound, meadowfoam delta-lactone, commercially available from The Fanning Corporation under the tradename Meadowlactone® and corresponding to formula (III):

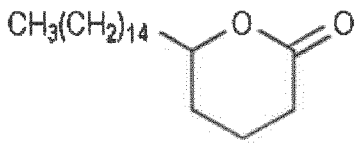

(III).

The heterocyclic compounds chosen from thiolactone compounds and their thiocarbonyl analogs, and lactam compounds and their thiocarbonyl analogs used in the method of the present invention are cyclic ester and cyclic amide compounds, respectively, comprising a heterocyclic ring. The heterocyclic compounds chosen from thiolactone compounds and their thiocarbonyl analogs, and lactam compounds and their thiocarbonyl analogs are represented by the general formula (I):

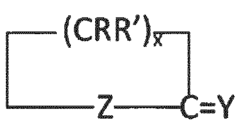

(I)

and wherein:

R or R', independently, is H; a hydrocarbon radical containing from 1 to 40 carbon atoms which may be saturated or unsaturated, linear or branched, substituted or unsubstituted; or a substituent other than H or said hydrocarbon radical, such as a hydroxy group, an amino group, a sulfhydryl group, an aryl group and a halogen;
X represents an integer of 1 or more, such as an integer from between 1 to 10 or such as from 1 to 8 or such as from 1 to 6;

Z is sulfur (S) or NR", wherein R" is H or a hydrocarbon radical containing from 1 to 40 carbon atoms which may be saturated or unsaturated, linear or branched, substituted or unsubstituted;
Y is oxygen (O) or sulfur (S); and
the heterocyclic ring is saturated or unsaturated.

More particularly, the heterocyclic compound chosen from thiolactone compounds and their thiocarbonyl analogs, and lactam compounds and their thiocarbonyl analogs used in the method of the present invention include, but are not limited, to those heterocyclic compounds chosen from thiolactone compounds and their thiocarbonyl analogs, and lactam compounds and their thiocarbonyl analogs comprising 3- to 8-membered rings (including the heteroatom on the heterocyclic ring and the carbonyl carbon).

The heterocyclic compound chosen from thiolactone compounds and their thiocarbonyl analogs, and lactam compounds and their thiocarbonyl analogs used in the method of the present invention may be chosen from α-thiolactone compounds and α-lactam compounds (3-membered ring), β-thiolactone compounds and β-lactam compounds (4-membered ring), γ-thiolactone compounds and γ-lactam compounds (5-membered ring), δ-thiolactone compounds and δ-lactam compounds (6-membered ring), and ε-thiolactone compounds and ε-lactam compounds (8-membered ring).

Preferably, compounds used in the method of the present invention corresponding to formula (I) are those where at least one of the R and R' groups is a hydrocarbon radical containing from 1 to 40 carbon atoms which may be saturated or unsaturated, linear or branched, substituted or unsubstituted, and x is at least 3.

Examples of the heterocyclic compound chosen from thiolactone compounds and their thiocarbonyl analogs used in the method of the present invention are Gamma-Thiobutyrolactone (dihydro-2(3H)-thiophenone or γ-Thiobutyrolactone), DL-Homocysteine thiolactone hydrochloride (3-aminodihydro-2(3H)-thophenone hydrochloride), L-Homocysteine tholactone hydrochloride ((3S)-3-aminodihydro-2(3H)-thiophenone hydrochloride), N-Acetyl-DL-Homocysteine thiolactone (N-(2-oxotetrahydro-3-thienyl)acetamide), N-Butyryl-DL-homocysteine thiolactone (N-(2-thioxotetrahydro-3-furanyl)hexanamide), Thilactomycin ((5R)-4-hydroxy-3.5-dimethyl-5-[(1E)-2-methyl-1,3-butadienyl]-2(5H)-thiophenone), 4-HYDROXY-3,5-DIMETHYL-5-[(1E)-2-METHYL-1,3-BUTADIENYL]-2(5h)-THIOPHENONE (4-hydroxy-3,5-dimethyl-5-[(1E)-2-methyl-1,3-butadienyl]-2(5H)-thiophenone, Kadethrin ((5-benzyl-3-furyl)methyl (1R,3S)-2,2-dimethyl-3-[(E)-(2-oxodihydro-3(2H)-thienylidene)methyl]cyclopropanecarboxylate).

In certain embodiments of the method of the present invention, the thiolactone compounds and their thiocarbonyl analogs are chosen from γ-Thiobutyrolactone, homocysteine thiolactone, and their derivatives.

Examples of the heterocyclic compound chosen from lactam compounds and their thiocarbonyl analogs used in the method of the present invention are 2-azetidinone, 2-pyrrolidinone, 2-piperidinone, 2-azocanone, 2-azepanone and their derivatives.

In certain embodiments of the method of the present invention, the lactam compounds and their thiocarbonyl analogs are chosen from ε-Caprolactam, oenantholactam and lauryllactam.

The at least one heterocyclic compound chosen from lactone compounds and their thiocarbonyl analogs, thiolactone compounds and their thiocarbonyl analogs, and lactam compounds and their thiocarbonyl analogs, used in the method of the present invention, is typically employed if an amount ranging from 0.01% to 99.99% by weight, preferably from 0.01% to 70% by weight, preferably from 0.05% to 50% by weight, more preferably from 0.1% to 40% by weight, even more preferably from 0.1% to 10% by weight, based on the total weight of the composition applied in the method, including all ranges and subranges therebetween.

The at least one heterocyclic compound chosen from γ-thiobutyrolactone, meadowfoam delta-lactone and mixtures thereof is typically employed in an amount ranging from 0.01% to 99.99% by weight, preferably from 0.01% to 70% by weight, preferably from 0.05% to 50% by weight, more preferably from 0.1% to 40% by weight, even more preferably from 0.1% to 10% by weight, based on the total weight of the composition of the present invention, including all ranges and subranges therebetween.

ALKOXYSILANE HAVING AT LEAST ONE AMINO SUBSTITUENT

The alkoxysilane having at least one amino substituent used in the method of the present invention has an amine group available to react with the heterocyclic compound chosen from lactone compounds and their thiocarbonyl analogs, thiolactone compounds and their thiocarbonyl analogs, and lactam compounds and their thiocarbonyl analogs used in the method of the present invention.

The alkoxysilane having at least one amino substituent used in the method of the present invention can also have at least one solubilizing functional group. As used herein, the term "at least one solubilizing functional group" means any functional chemical group facilitating the bringing into solution of the alkoxysilane in the solvent or in a combination of solvents of the composition, for example, in solvents chosen from water, water-alcoholic mixtures, organic solvents, polar solvents and non-polar solvents.

Suitable solubilizing functional groups for use in the method of the present invention include, but are not limited

to, primary, secondary, and tertiary amine, aromatic amine, alcohol, carboxylic acid, sulfonic acid, anhydride, carbamate, urea, guanidine, aldehyde, ester, amide, epoxy, pyrrole, dihydroimidazole, gluconamide, pyridyle, and polyether groups.

The at least one alkoxysilane having at least one amino substituent used in the method of the present invention may comprise at least one silicon atom.

The at least one alkoxysilane having at least one amino substituent used in the method of the present invention may, in at least one embodiment, comprise two or three alkoxy functional groups. In another embodiment of the method, the alkoxy functional groups are chosen from methoxy and ethoxy functional groups.

According to one embodiment of the method of the present invention, the at least one alkoxysilane having at least one amino substituent is chosen from compounds of formula (III):

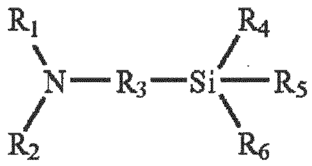

(III)

wherein:

- $R_4$ is chosen from OR' groups;
- $R_5$ is chosen from OR" groups;
- $R_6$ is chosen from OR'" groups,;
- $R_1$, $R_2$ are chosen from hydrogen;
- $R_3$, R', R", R'", which may be identical or different, are chosen from linear and branched, saturated and unsaturated hydrocarbon groups, optionally bearing at least one additional chemical group, wherein R', R", and R'" may also be chosen from hydrogen.

In at least one embodiment of the method, the R', R", and R'" groups are chosen from $C_1$-$C_{12}$ alkyl, $C_6$-$C_{14}$ aryl, $C_1$-$C_8$ alkyl-$C_6$-$C_{14}$ aryl, and $C_6$-$C_{14}$ aryl-$C_1$-$C_8$-alkyl radicals.

Particularly preferred alkoxysilanes having at least one amino substituent used in the method of the present invention include alkoxysilanes comprising a silicone atom. Possible examples of useful alkoxysilanes used in the method of the present invention include aminoalkyltrialkoxysilanes such as 3-aminopropyltriethoxysilane, as described in French Patent Application No. FR 2 789 896.

In another embodiment, the alkoxysilanes used in the method of the present invention may be alkoxysilanes which carry a group having a cosmetic functional group, such as aromatic nitro dyes or anthraquinone, napthoquinone, benzoquinone, azo, xanthene, triarylmethane, azine, indoaniline, indophenolic or indoamine dyes; groups having a reductive effect, such as thiol groups, sulphinic acid or sulphinic salt, it being possible for these alkoxysilanes to carry a solubilizing non-hydrolysable group such as amino groups, carboxylic acids, sulphonic acids, sulphates, quaternary ammoniums, polyalcohols, polyether and phosphates. One possible example includes aminopropyl-N-(4,2-dinitrophenyl)aminopropyldiethoxysilane. Compounds of this kind are described, for example, in Patent Application EP 1 216 023.

The alkoxysilanes used in the method of the present invention may be amino aryl alkoxysilanes. Possible examples include but are not limited to the following compounds:

3-(m-aminophenoxy)propyltrimethoxysilane, of the formula (IV):

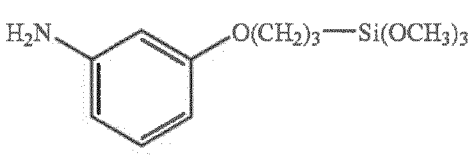

(IV)

provided by GELEST,

p-aminophenyltrimethoxysilane, of formula (V):

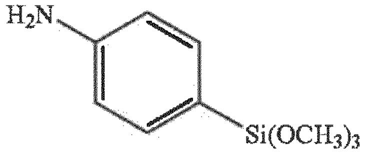

(V)

provided by GELEST, and
N-(2-aminoethylaminomethyl)phenethyltrimethoxysilane, of the formula (VI) :

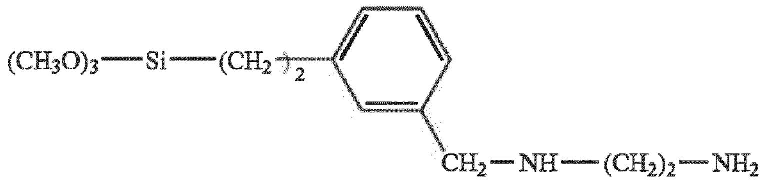

(VI)

provided by GELEST.

In another embodiment the at least one alkoxysilane having at least one amino substituent used in the method of the present invention is a trialkoxysilane.

In another embodiment, the at least one alkoxysilane having at least one amino substituent used in the method of the present invention is a trialkoxysilane having one amino substituent.

A particularly preferred at least one alkoxysilane having at least one amino substituent used in the method of the present invention is a y-aminopropyltriethoxysilane, also known as 3-aminopropyltriethoxysilane, commercially available under the tradename, KBE-903™, from Shin-Etsu, and also under the tradename, Silsoft® A-1100, from Momentive Performance Materials.

The at least one alkoxysilane having at least one amino substituent used in the method of the present invention is typically employed in an amount ranging from 0.01% to 99.99% by weight, preferably from 0.01% to 70% by weight, preferably from 0.05% to 50% by weight, more preferably from 0.1% to 40% by weight, even more preferably from 0.1% to 10% by weight, based on the total weight of the composition applied in the method, including all ranges and subranges therebetween.

3-aminopropyltriethoxysilane is typically employed in an amount ranging from 0.01% to 99.99% by weight, preferably from 0.01% to 70% by weight, preferably from 0.05% to 50% by weight, more preferably from 0.1% to 40% by weight, even more preferable from 0.1% to 10% by weight, based on the total weight of the composition, including all ranges and subranges therebetween.

The at least one heterocyclic compound chosen from γ-thiobutyrolactone, meadowfoam delta-lactone and mixtures thereof and 3- aminopropyltriethoxysilane may be present in the compositions of the present invention in a combined amount of from 0.01% to 50% by weight, such as from 0.05% to 40% by weight, such as from 0.1% to 30% by weight, such as from 0.25% to 20% by weight, or such as from 0.5% to 10% by weight, based on the total weight of the compositions of the present invention, including all ranges and subranges therebetween.

REACTION PRODUCT

The combination of the at least one heterocyclic compound chosen from lactone compounds and their thiocarbonyl analogs, thiolactone compounds and their thiocarbonyl analogs, and lactam compounds and their thiocarbonyl analogs with the at least one alkoxysilane having at least one amino substituent used in the method of the present invention may result in the formation of a reaction product.

The combination of the at least one heterocyclic compound chosen from γ-thiobutyrolactone, meadowfoam delta-lactone and mixtures thereof with 3-aminopropyltriethoxysilane used in the composition off the present invention may result in the formation of a reaction product.

Although not wanting to be bound by any particular theory, it is believed that the amine group of the 3-aminopropyltriethoxysilane or of the at least one alkoxysilane having at least one amino substituent reacts with the carbonyl group on the heterocyclic ring of the at least one heterocyclic compound, resulting in the opening of the heterocyclic ring and in the formation of the reaction product of the present invention wherein the reaction product is of the amide type or thioamide type.

It is not necessary for all amine groups and all carbonyl groups to react with each other to form the reaction

product. Rather, it is possible that the compositions applied in the method of the present invention may contain free alkoxysilane having at least one amino substituent and/or free heterocyclic compound chosen from lactone compounds and their thiocarbonyl analogs, thiolactone compounds and their thiocarbonyl analogs, and lactam compounds and their thiocarbonyl analogs, or that the composition of the present invention may contain free 3-aminopropyltriethoxysilane and/or free heterocyclic compound chosen from γ-thiobutyrolactone, meadowfoam delta-lactone and mixtures thereof, in addition to the reaction product.

The appropriate amount of the heterocyclic compound to react with the alkoxysilane to obtain the reaction product of the present invention can be easily determined.

The amount of the at least one alkoxysilane having at least one amino substituent used in the method or of 3-aminopropyltriethoxysilane used in the composition to react with said at least one heterocyclic compound typically ranges from 0.01% to 99.99% by weight, preferably from 0.01% to 70% by weight, preferably from 0.05% to 50% by weight, more preferably from 0.1% to 40% by weight, even more preferably from 0.1% to 10% by weight, based on the total weight of the reaction product, including all ranges and subranges therebetween.

The amount of the at least one heterocyclic compound to react with said at least one alkoxysilane having at least one amino substituent in the method, or with 3-aminopropyltriethoxysilane in the composition typically ranges from 0.01% to 99.99% by weight, preferably from 0.01% to 70% by weight, preferably from 0.05% to 50% by weight, more preferably from 0.1% to 40% by weight, even more preferably from 0.1% to 10% by weight, based on the total weight of the reaction product, including all ranges and subranges therebetween.

In preferred embodiments of the present invention, the molar ratio of the at least one heterocyclic compound to the at least one alkoxysilane having at least one amino substituent in the method, or to 3-aminopropyltriethoxysilane in the composition ranges from between 10:1 to 1:10, such as from 8:1 to 1:8, such as from between 5:1 to 1:5, such as from between-3:1 to 1:3, such as from between 2:1 to 1:2, including all ranges and subranges therebetween.

In particularly preferred embodiments, the molar ratio of the at least one heterocyclic compound to the at least one alkoxysilane having at least one amino substituent in the method, or to 3-aminopropyltriethoxysilane in the composition is 1:1 or 1:2 or 2:1.

According to preferred embodiments of the method of the present invention, when at least one of the carbon atoms on the heterocyclic ring of the at least one heterocyclic compound chosen from lactone compounds and their thiocarbonyl analogs, thiolactone compounds and their thiocarbonyl analogs, and lactam compounds and their thiocarbonyl analogs is substituted with a hydrocarbon radical containing at least 6 carbon atoms, the at least one alkoxysilane having at least one amino substituent does not require a substituent comprising at least one hydrocarbon radical containing at least 6 carbon atoms.

According to other preferred embodiments of the method of the present invention, when none of the carbon atoms on the heterocyclic ring of the at least one heterocyclic compound chosen from lactone compounds and their thiocarbonyl analogs, thiolactone compounds and their thiocarbonyl analogs, and lactam compounds and their thiocarbonyl analogs is substituted with a hydrocarbon radical containing at least 6 carbon atoms, the at least one alkoxysilane having at least one amino substituent contains a substituent comprising at least one hydrocarbon radical containing at least 6 carbon atoms.

In preferred embodiments of the method of the present invention, the reaction product has at least one substituent comprising a hydrocarbon radical containing at least 6 carbon atoms.

According to particularly preferred embodiments of the method, when the at least one alkoxysilane having at least one amino substituent is chosen from 3-aminopropyltriethoxysilane, the reaction product of the present invention is of the monoamide type.

Without being bound to any particular theory, it is also believed that other functional groups on the reaction product of the heterocyclic compound chosen from lactone compounds and their thiocarbonyl analogs, thiolactone compounds and their thiocarbonyl analogs, and lactam compounds and their thiocarbonyl analogs and the alkoxysilane having at least one amino substituent may further react or interact with the carbonyl group of any unreacted heterocyclic compound chosen from lactone compounds and their thiocarbonyl analogs, thiolactone compounds and their thiocarbonyl analogs, and lactam compounds and their thiocarbonyl analogs.

According to preferred embodiments, the heterocyclic compound and the alkoxysilane are mixed together at room temperature under anhydrous conditions. The mixture is then preferably heated beyond the melting point of the alkoxysilane and/or of the heterocyclic compound, or of the mixture of the alkoxysilane and the heterocyclic compound, typically up to 100°C or 110°C or 120°C for at least 30 minutes, or for at least 60 minutes, or for at least 120 minutes, including all time intervals therein, to form the reaction product of the present invention.

The reaction product of the present invention may be in the form of a solid or in the form of a liquid.

In preferred embodiments, the reaction product of the present invention is combined with at least one carrier to form a composition such as a cosmetic, personal care or dermatological composition.

The reaction product of the present invention is combined with compositions comprising at least one benefit agent.

In other preferred embodiments, the heterocyclic compound and the alkoxysilane are mixed together at room temperature under anhydrous conditions in the presence of at least one anhydrous, non-reactive solvent. The reaction product may be formed at room temperature or the heterocyclic compound-alkoxysilane mixture may heated beyond the melting point of the alkoxysilane and/or of the heterocyclic compound, or of the mixture of the alkoxysilane and the heterocyclic compound, typically up to 100°C or 110°C or 120°C for at least 30 minutes, or for at least 60 minutes, or for at least 120 minutes, including all time intervals therein, to form the reaction product of the present invention.

The anhydrous, non-reactive solvent in which the reaction product can be prepared may be chosen from oils, organic solvents, esters, and silicones and may include, but is not limited to, hydrocarbon-based compounds such as isododecane, isohexadecane, paraffin, isoparaffin, and mineral oil, and silicone oils such as dimethicone.

In some embodiments, the reaction product that is prepared in at least one anhydrous, non-reactive solvent may be in a ready-to-use form and may be used as a cosmetic, personal care or dermatological composition.

In other embodiments, the reaction product that is prepared in at least one anhydrous, non-reactive solvent is combined with at least one carrier or with compositions comprising at least one benefit agent.

It was surprisingly and unexpectedly discovered that the compositions containing the reaction product of the present invention are water resistant and provide water resistant properties and long lasting and durable hydrophobicity to the surface of keratinous substrates. Moreover, said compositions are stable and are capable of carrying various types of ingredients, such as benefit agents.

It was also surprisingly and unexpectedly discovered that the compositions containing the reaction product of the present invention have improved water resistance properties when applied onto keratinous substrates.

The amount of the reaction product typically ranges from 0.01% to 50% by weight, such as from 0.05% to 40% by weight, such as from 0.1% to 30% by weight, such as from 0.25% to 20% by weight, or such as from 0.5% to 10% by weight, based on the total weight of the compositions of the present invention, including all ranges and subranges therebetween.

The presence of a monoacid is not required in the compositions, including the reaction product, of the present invention, in order for said compositions to be water resistant. Thus, according to a particularly preferred embodiment, the composition of the invention does not contain any monoacid.

## CARRIER

The at least one carrier of the present invention may be an anhydrous carrier or an emulsion carrier or an aqueous carrier or aqueous-alcoholic carrier or an alcoholic carrier or a solid carrier or a vaporizable carrier. When it is an emulsion, it may be an oil-in-water emulsion, water-in-oil emulsion, silicone-in-water emulsion, or water-in-silicone emulsion.

In preferred embodiments of the present invention, the at least one carrier comprises at least one compound chosen from water, oils, alcohols, organic solvents, esters, silicones, waxes, and mixtures thereof.

In other preferred embodiments, the at least one carrier comprises and/or includes either the at least one heterocyclic compound, or 3-aminopropyltriethoxysilane whichever one is in excess and remains unreacted after the reaction product of the present invention is formed.

Suitable oils that may comprise the carrier include, but are not limited to, mineral oils (paraffin); plant oils (sweet almond oil, macadamia oil, grapeseed oil or jojoba oil); synthetic oils, for instance perhydrosqualene, fatty alcohols, fatty acids or fatty esters (for instance the $C_{12}$-$C_{15}$ alkyl benzoate sold under the trade name Finsolv® TN, commercially available from Innospec or Tegosoft® TN, commercially available from Evonik Goldschmidt, octyl palmitate, isopropyl lanolate and triglycerides, including capric/caprylic acid triglycerides), oxyethylenated or oxypropylenated fatty esters and ethers; silicone oils (cyclomethicone and polydimethylsiloxanes, or PDMS) or fluoro oils, and polyalkylenes.

Other oils that may comprise the carrier may include for example: silicone oils, for instance volatile or non-volatile polymethylsiloxanes (PDMS) with a linear or cyclic silicone chain, which are liquid or pasty at room temperature, especially cyclopolydimethylsiloxanes (cyclomethicones) such as cyclohexasiloxane; polydimethyl-siloxanes comprising alkyl, alkoxy or phenyl groups, which are pendent or at the end of a silicone chain, these groups containing from 2 to 24 carbon atoms; phenyl silicones, for instance phenyl trimethicones, phenyl dimethicones, phenyltrimethylsiloxydiphenyl-siloxanes, diphenyl dimethicones, diphenylmethyl-diphenyltrisiloxanes or 2-phenylethyl trimethylsiloxy silicates, and polymethylphenylsiloxanes; mixtures thereof. Particularly representative of such oils are volatile silicone oils, such as cyclomethicones.

Other suitable oils include, but are not limited to, volatile hydrocarbon-based oils such as, for example, volatile hydrocarbon oils having from 8 to 16 carbon atoms and their mixtures and in particular branched $C_8$ to $C_{16}$ alkanes such as $C_8$ to $C_{16}$ isoalkanes (also known as isoparaffins), isododecane, isodecane, isohexadecane, and for example, the oils sold under the trade names of Isopar™ or Permethyl®, and their mixtures.

Examples of other oils also include branched and unbranched hydrocarbons and hydrocarbon waxes including polyolefins, in particular petrolatum, paraffin oil, squalane, squalene, hydrogenated polyisobutene, hydrogenated

polydecene, polybutene, pentahydrosqualene, and mixtures thereof.

Among the alcohols and organic solvents that may be mentioned are lower alcohols such as ethanol, fatty alcohols and polyols. The fatty alcohols may be chosen from those of the formula R-OH where R represents a linear or branched higher fatty acid residue containing from 8 to 40 carbon atoms. The polyols may be chosen from glycols and glycol ethers, for instance glycerol, ethylene glycol, propylene glycol, butylene glycol, dipropylene glycol or diethylene glycol.

Suitable esters that may comprise the carrier include, but are not limited to, esters of formula $R_1COOR_2$ in which $R_1$ represents a linear or branched higher fatty acid residue containing from 1 to 40 carbon atoms, including from 7 to 19 carbon atoms, and $R_2$ represents a branched hydrocarbon-based chain containing from 1 to 40 carbon atoms, including from 3 to 20 carbon atoms, and also including, for example, octyldodecyl neopentanoate, Purcellin oil (cetostearyl octanoate), isononyl isononanoate, $C_{12}$ to $C_{15}$ alkyl benzoate, isopropyl myristate, 2-ethylhexyl palmitate, and octanoates, decanoates or ricinoleates of alcohols or of polyalcohols; hydroxylated esters, for instance isostearyl lactate or diisostearyl malate, and pentaerythritol esters.

Other suitable esters that may comprise the carrier include polyesters, alkoxylated esters, and alkoxylated polyesters.

Suitable silicones that may comprise the carrier include, but are not limited to, the silicone oils described above and other silicones such as non-volatile silicones such as dimethicone fluids having viscosity values of equal to or greater than 300 cst, and pentaphenyldimethicone, also known as trimethyl pentaphenyl trisiloxane, commercially available from Dow Corning under the tradename Dow Corning® 555.

Suitable waxes that may comprise the carrier include, but are not limited to, those of natural origin, such as beeswax, carnauba wax, candelilla wax, ouricoury wax, Japan wax, cork fibre wax or sugar cane wax, rice wax, montan wax, paraffin wax, lignite wax or microcrystalline wax, ceresin or ozokerite, palm kernel glycerides/hydrogenated palm glycerides and hydrogenated oils such as hydrogenated castor oil or jojoba oil; synthetic waxes such as the polyethylene waxes obtained from the polymerization or copolymerization of ethylene, and Fischer-Tropsch® waxes, or else esters of fatty acids, such as octacosanyl stearate, glycerides which are concrete at 30°C, for example at 45°C, silicone waxes, such as alkyl- or alkoxydimethicones having an alkyl or alkoxy chain ranging from 10 to 45 carbon atoms, poly(di)methylsiloxane esters which are solid at 30°C. and whose ester chain comprising at least 10 carbon atoms, or else di(1,1,1-trimethylolpropane) tetrastearate, which is sold or manufactured by Heterene under the name HEST® 2T-4S, and mixtures thereof.

The at least one carrier of the present invention may comprise a non-reactive solvent.

In preferred embodiments, the at least one carrier of the present invention may comprise a cosmetically or physiologically acceptable medium that is non toxic, wherein the compositions can be applied onto keratinous substrates such the skin, lips, hair, scalp, lashes, brows, nails or any other cutaneous region of the body. The cosmetically or physiologically acceptable medium may comprise one or more of the oils, solvents and carriers mentioned above.

In preferred embodiments of the present invention, the cosmetically or physiologically acceptable medium comprises at least one compound chosen from water, oils, alcohols, organic solvents, esters, silicones, waxes, and mixtures thereof.

The carrier can be employed in an amount of from 0.01% to 99.98% by weight, such as from 1% to 99% by weight, or such as from 2% to 90% by weight, or such as from 5% to 80% by weight, and from 10% to 70% by weight, based on the total weight of the composition.

BENEFIT AGENT

The compositions of the present invention further comprise at least one benefit agent chosen from sunscreen agents, cosmetically and dermatologically active agents, humectants and moisturizing agents, colorants, hair straightening/relaxing agents, film forming agents, shine agents, conditioning agents, reducing agents, emollients, vitamins, antidandruff agents, plant extracts, antiperspirants, and pharmaceutical agents.

Representative sunscreen agents may be chosen from organic and inorganic sunscreens or UV filters.

The organic sunscreen agents are selected from water-soluble organic screening agents, fat-soluble organic screening agents or agents which are insoluble in the solvents presently included in suntan products, and mixtures thereof.

The organic sunscreen agents are especially selected from cinnamic derivatives; anthranilates; salicylic derivatives; dibenzoylmethane derivatives; camphor derivatives; benzophenone derivatives; beta, beta -diphenylacrylate derivatives; triazine derivatives; benzotriazole derivatives; benzalmalonate derivatives; benzimidazole derivatives; imidazolines; bis-benzazolyl derivatives; p-aminobenzoic acid (PABA) derivatives; methylenebis(hydroxyphenylbenzotriazole) derivatives; benzoxazole derivatives; screening polymers and screening silicones; alpha -alkylstyrene-derived dimers; 4,4-diarylbutadienes; merocyanin derivatives; and mixtures thereof.

Examples of complementary organic photoprotective agents include those denoted hereinbelow under their INCI name:

Cinnamic Derivatives:

Ethylhexyl Methoxycinnamate marketed in particular under the trademark "Parsol MCX®" by DSM Nutritional Products, Inc., Isopropyl Methoxycinnamate, Isoamyl p-Methoxycinnamate marketed under the trademark "Neo Heliopan E 1000®" by Symrise, DEA Methoxycinnamate, Diisopropyl Methylcinnamate, Glyceryl Ethylhexanoate Dimethoxycinnamate.

Dibenzoylmethane Derivatives: [Butyl Methoxydibenzoylmethane marketed especially under the trademark "Parsol 1789®" by DSM Nutritional Products, Inc., Isopropyl Dibenzoylmethane.

Para-Aminobenzoic Acid Derivatives: PABA, Ethyl PABA, Ethyl Dihydroxypropyl PABA, Ethylhexyl Dimethyl PABA marketed in particular under the trademark "Escalol 507®" by ISP, Glyceryl PABA, PEG-25 PABA marketed under the trademark "Uvinul P25®" by BASF.

Salicylic Derivatives: Homosalate marketed under the trademark "Eusolex HMS®" by Merck KGaA /EMD Chemicals, Inc. and EMD Chemicals Inc, Ethylhexyl Salicylate marketed under the trademark "Neo Heliopan OS®" by Symrise, Dipropylene Glycol Salicylate marketed under the trademark "Dipsal™" by Lubrizol Advanced Materials, Inc., TEA Salicylate marketed under the trademark "Neo Heliopan® TS" by Symrise. beta, beta - Diphenylacrylate Derivatives:

Octocrylene marketed in particular under the trademark "Uvinul N539T®" by BASF, Etocrylene marketed in particular under the trademark "Uvinul® N35" by BASF.

Benzophenone Derivatives: Benzophenone-1 marketed under the trademark "Uvinul® 400" by BASF, Benzophenone-2 marketed under the trademark "Uvinul® D50" by BASF, Benzophenone-3 or Oxybenzone marketed under the trademark "Uvinul® M40" by BASF, Benzophenone-4 marketed under the trademark "Uvinul® MS40" by BASF, Benzophenone-5, Benzophenone-6 marketed under the trademark "Helisorb® 11" by Norquay, Benzophenone-8, Benzophenone-9, Benzophenone-12, n-Hexyl 2-(4-diethylamino-2-hydroxybenzoyl)benzoate marketed under the trademark "Uvinul® A+" by BASF.

Benzylidenecamphor Derivatives: 3-Benzylidenecamphor manufactured under the trademark "Mexoryl™ SD" by Chimex, 4-Methylbenzylidenecamphor marketed under the trademark "Eusolex® 6300" by Merck, Benzylidene Camphor Sulfonic acid manufactured under the trademark "Mexoryl™ SL" by Chimex, Camphor Benzalkonium Methosulfate manufactured under the trademark "Mexoryl™ SO" by Chimex, Terephthalylidene Dicamphor Sulfonic acid manufactured under the trademark "Mexoryl™ SX" by Chimex, Polyacrylamidomethyl Benzylidene Camphor manufactured under the trademark "Mexoryl™ SW" by Chimex.

Phenylbenzimidazole Derivatives: Phenylbenzimidazole Sulfonic acid marketed in particular under the trademark "Eusolex® 232" by Merck and EMD INC., Disodium Phenyl Dibenzimidazole Tetrasulfonate marketed under the trademark "Neo Heliopan® AP" by Symrise.

Phenylbenzotriazole Derivatives: Drometrizole Trisiloxane, Methylene bis(Benzotriazolyl) Tetramethylbutylphenol, or in micronized form as an aqueous dispersion under the trademark "Tinosorb® M" by BASF.

Triazine Derivatives: bis-Ethylhexyloxyphenol Methoxyphenyl Triazine marketed under the trademark "Tinosorb® S" by BASF, Ethylhexyl Triazone marketed in particular under the trademark "Uvinul® T150" by BASF, Diethylhexyl Butamido Triazone marketed under the trademark "Uvasorb® HEB" by 3V Group, 2,4,6-Tris(dineopentyl 4'-aminobenzalmalonate)-s-triazine, 2,4,6-Tris(diisobutyl 4'-aminobenzalmalonate)-s-triazine, 2,4-Bis(n-butyl 4'-aminobenzoate)-6-(aminopropyltrisiloxane)-s-triazine, 2,4-Bis(dineopentyl 4'-aminobenzalmalonate)-6-(n-butyl 4'-aminobenzoate)-s-triazine, triazine agents, especially 2,4,6-tris(biphenyl-1,3,5-triazines (in particular 2,4,6-tris(biphenyl-4-yl)-1,3,5-triazine and 2,4,6-tris(terphenyl)-1,3,5-triazine.

Anthranilic Derivatives: Menthyl anthranilate marketed under the trademark "Neo Heliopan® MA" by Symrise.

Imidazoline Derivatives: Ethylhexyl Dimethoxybenzylidene Dioxoimidazoline Propionate.

Benzalmalonate Derivatives: Polyorganosiloxane containing benzalmalonate functions, for instance Polysilicone-15, marketed under the trademark "Parsol® SLX" by DSM Nutritional Products, Inc..

4,4-Diarylbutadiene Derivatives: 1,1-Dicarboxy(2,2'-dimethylpropyl)-4,4-diphenylbutadiene.

Benzoxazole derivatives: 2,4-Bis[5-(1-dimethylpropyl)benzoxazol-2-yl(4-phenyl)imino]-6-(2-ethylhexyl)imino-1,3,5-triazine marketed under the trademark Uvasorb K 2A by Sigma 3V, and mixtures thereof.

The preferred organic sunscreen agents are selected from: Ethylhexyl Methoxycinnamate, Ethylhexyl Salicylate, Homosalate, Butyl Methoxydibenzoylmethane, Octocrylene, Phenylbenzimidazole Sulfonic Acid, Benzophenone-3, Benzophenone-4, Benzophenone-5, n-Hexyl 2-(4-diethylamino-2-hydroxybenzoyl)benzoate, 4-Methylbenzylidene camphor, Terephthalylidene Dicamphor Sulfonic Acid, Disodium Phenyl Dibenzimidazole Tetrasulfonate, Methylene bis-Benzotriazolyl Tetramethylbutylphenol, Bis-Ethylhexyloxyphenol Methoxyphenyl Triazine, Ethylhexyl Triazone, Diethylhexyl Butamido Triazone, 2,4,6-Tris(dineopentyl 4'-aminobenzalmalonate)-s-triazine, 2,4,6-Tris(diisobutyl 4'-aminobenzalmalonate)-s-triazine, 2,4-Bis(n-butyl 4'-aminobenzoate)-6-(aminopropyltrisiloxane)-s-triazine, 2,4-Bis(dineopentyl 4'-aminobenzalmalonate)-6-(n-butyl 4'-aminobenzoate)-s-triazine, 2,4,6-Tris(biphenyl-4-yl)-1,3,5-triazine, 2,4,6-Tris(terphenyl)-1,3,5-triazine, Drometrizole Trisiloxane, Polysilicone-15, 1,1-Dicarboxy(2,2'-dimethylpropyl)-4,4-diphenylbutadiene, 2,4-Bis[5-1-(dimethylpropyl)benzoxazol-2-yl(4-phenyl)imino]-6-(2-ethylhexyl)-imino-1,3,5-triazine, and mixtures

thereof.

Examples of inorganic sunscreen agents or UV filters include, but are not limited to, metal oxide pigments which may be chosen from zinc oxide, titanium oxide, iron oxide, zirconium oxide, cerium oxide, and mixtures thereof.

The metal oxide pigments may be coated or uncoated.

The coated pigments are pigments that have undergone one or more surface treatments of chemical, electronic, mechanochemical and/or mechanical nature with compounds such as amino acids, beeswax, fatty acids, fatty alcohols, anionic surfactants, lecithins, sodium potassium, zinc, iron or aluminum salts of fatty acids, metal alkoxides (of titanium or of aluminum), polyethylene, silicones, proteins (collagen, elastin), alkanolamines, silicon oxides, metal oxides or sodium hexametaphosphate.

The sunscreen agents of the present invention may be employed in an amount of from 0.1 % to 40 % by weight, such as from 0.5 % to 30 % by weight, such as from 1 % to 25 % by weight, based on the total weight of the composition.

Representative cosmetically and dermatologically active agents include, but are not limited to:

- antipollution agents and/or free-radical scavengers;
- depigmenting agents and/or propigmenting agents;
- self-tanning agents;
- anti-acne agents;
- anti-aging agents;
- antiglycation agents;
- NO-synthase inhibitors;
- agents for stimulating the synthesis of dermal or epidermal macromolecules and/or for preventing their degradation;
- agents for stimulating fibroblast proliferation;
- agents for stimulating keratinocyte proliferation;
- muscle relaxants;
- tensioning agents;
- desquamating and exfoliating agents;
- moisturizers and humectants;
- anti-inflammatory agents;
- agents acting on the energy metabolism of cells;
- insect repellants;
- substance P or CGRP antagonists.

Suitable examples of humectants and moisturizing agents include, but are not limited to urea, hydroxyethyl urea, polyols such as glycerin, and glycosaminoglycans (GAGs). Suitable examples of glycosaminoglycans are hyaluronic acid or hyaluronan (HA), heparan sulfate (HS), heparin (HP), chondroitin, chondroitin sulfate (CS), chondroitin 4-sulfate or chondroitin sulfate A (CSA), chondroitin 6-sulfate or chondroitin sulfate C (CSC), dermatan sulfate or chondroitin sulfate B (CSB) and keratan sulfate (KS).

Acceptable colorants include, but are not limited to pigments, dyes, such as liposoluble dyes, nacreous pigments, pearling agents, direct dyes and oxidation dyes.

Representative liposoluble dyes which may be used according to the present invention include Sudan Red, DC Red 17, DC Green 6, β-carotene, soybean oil, Sudan Brown, DC Yellow 11, DC Violet 2, DC Orange 5, annatto, and quinoline yellow.

Representative nacreous pigments include white nacreous pigments such as mica coated with titanium or with bismuth oxychloride, colored nacreous pigments such as titanium mica with iron oxides, titanium mica with ferric blue or chromium oxide, titanium mica with an organic pigment chosen from those mentioned above, and nacreous pigments based on bismuth oxychloride.

Representative pigments include white, colored, inorganic, organic, polymeric, nonpolymeric, coated and uncoated pigments. Representative examples of mineral pigments include titanium dioxide, optionally surface-treated, zirconium oxide, zinc oxide, cerium oxide, iron oxides, chromium oxides, manganese violet, ultramarine blue, chromium hydrate, and ferric blue. Representative examples of organic pigments include carbon black, pigments of D & C type, and lakes based on cochineal carmine, barium.

The direct dyes and oxidation dyes which may be used in the present invention are those dyes employed to color hair. Representative oxidation dyes include, but are not limited to para-phenylenediamines, bis(phenyl)alkylenediamines, para-aminophenols, ortho-aminophenols and heterocyclic bases, and the addition salts thereof. Representative direct dyes include, but are not limited to, azo, methane, carbonyl, azine, nitro (hetero)aryl, tri(hetero)arylmethane, porphyrin, phthalocyanin direct dyes, and natural direct dyes.

The hair straightening/relaxing agents and reducing agents which may be used in the present invention are those compounds which are employed to permanently or temporarily change the shape of the hair such as, for example,

hydroxide and non-hydroxide bases, amine-based compounds, and thiol-based agents.

The composition(s) of the present invention may also comprise additives, for instance those chosen from the non-exhaustive list such as rheology-modifying agents, film-forming agents, surfactants, sequestering agents, softeners, antifoams, basifying agents, gelling agents, wetting agents, thickening agents, spreading agents, dispersants, plasticizers, preservatives, pigments, mineral fillers, clays, colloidal minerals, nacres, nacreous agents, fragrances, peptizers, preserving agents, pH adjusters, fixing or non-fixing polymers, silicones, mineral, organic or plant oils, plant extracts, oxyethylenated or non-oxyethylenated waxes, paraffins, fatty acids, and the like.

According to some embodiments of the present invention, the compositions of the present invention are anhydrous.

In the event that the composition of the present invention includes water, the composition may comprise water in an amount of from 1% to 90% water, more preferably from 5% to 75% water, and more preferably from 15% to 50% water by weight with respect to the total weight of the composition, including all ranges and subranges therebetween.

The compositions of the present invention may be in the form of a simple or complex emulsion (oil-in-water (o/w), water-in-oil (w/o), silicone-in-water and / or water-in-silicone emulsion types) such as a cream or a milk, in the form of a gel or a cream-gel, or in the form of a lotion, a powder or a solid tube, and may optionally be packaged as an aerosol and may be in the form of a mousse or a spray. The mousse or spray may contain propellants such as for example, the hydrofluorinated compounds dichlorodifluoromethane, difluoroethane, dimethyl ether, isobutane, n-butane, propane or trichlorofluoromethane.

The emulsions of the present invention will generally contain at least one emulsifier chosen from amphoteric, anionic, cationic and nonionic emulsifiers, which are used alone or as a mixture. The emulsifiers are appropriately chosen according to the emulsion to be obtained.

As emulsifiers that may be used for the preparation of the W/O emulsions, examples that may be mentioned include sorbitan, glycerol or sugar alkyl esters or ethers; silicone surfactants, for instance dimethicone copolyols, such as the mixture of cyclomethicone and of dimethicone copolyol, sold under the tradename Dow Corning® DC 5225 C by the company Dow Corning, and alkyldimethicone copolyols such as laurylmethicone copolyol sold under the tradename Dow Corning® 5200 Formulation Aid by the company Dow Corning; cetyldimethicone copolyol, such as the product sold under the name Abil® EM 90R by the company Goldschmidt, and the mixture of cetyldimethicone copolyol, of polyglyceryl isostearate (4 mol) and of hexyl laurate, sold under the name Abil® WE 09 by the company Goldschmidt. One or more co-emulsifiers may also be added thereto, which may be chosen advantageously from the group comprising polyol alkyl esters. Polyol alkyl esters that may especially be mentioned include glycerol and/or sorbitan esters, for example polyglyceryl isostearate, such as the product sold under the name Isolan® GI 34 by the company Goldschmidt, sorbitan isostearate, such as the product sold under the name Arlacel 987 by the company ICI, sorbitan glyceryl isostearate, such as the product sold under the name Arlacel™ 986 by the company ICI, and mixtures thereof.

For the O/W emulsions, examples of emulsifiers that may be mentioned include nonionic emulsifiers such as oxyalkylenated (more particularly polyoxyethylenated) fatty acid esters of glycerol; oxyalkylenated fatty acid esters of sorbitan; oxyalkylenated (oxyethylenated and/or oxypropylenated) fatty acid esters; oxyalkylenated (oxyethylenated and/or oxypropylenated) fatty alkyl ethers; sugar esters, for instance sucrose stearate; fatty alkyl ethers of sugars, especially polyalkylglucosides (APG) such as decylglucoside and laurylglucoside sold, for example, by the company Henkel under the respective names Plantaren® 2000 and Plantaren® 1200, cetostearyl glucoside optionally as a mixture with cetostearyl alcohol, sold, for example, under the name Montanov™ 68 by the company SEPPIC, under the name Tego® Care CG90 by the company Goldschmidt and under the name Emulgade® KE3302 by the company Henkel, and also arachidyl glucoside, for example in the form of a mixture of arachidyl alcohol, behenyl alcohol and arachidyl glucoside, sold under the name Montanov® 202 by the company SEPPIC. According to one particular embodiment of the invention, the mixture of the alkylpolyglucoside as defined above with the corresponding fatty alcohol may be in the form of a self-emulsifying composition as described, for example, in document WO-A-92/06778.

In another embodiment of the invention, the subject compositions are formulated as water-in-silicone (W/Si) or silicone-in-water (Si/W) emulsions in which the continuous oily phase comprises at least one silicone oil. When the compositions of the invention are formulated as water-in-silicone emulsions, the silicone oils are preferably present in a proportion of at least 5 percent and preferably ranging from 10 percent to 45 percent by weight with respect to the total weight of the emulsion. The fatty phase of the water-in-oil emulsions according to the invention can additionally comprise one or more hydrocarbon-comprising oil(s) in a proportion preferably ranging up to 40 percent by weight with respect to the total weight of the fatty phase of the emulsion.

For the W/Si emulsions, examples of emulsifiers generally include polyether-modified silicones having a long chain of dimethyl siloxane units which carry polyethoxy-polypropoxy units in the chain and at the ends. Examples include cyclopentasiloxane PEG/PPG-18/18 dimethicone, PEG-12 Dimethicone, and PEG/PPG-19/19 Dimethicone sold by Dow Corning under the name Dow Corning® BY 11-030.

In accordance with preferred embodiments, the water resistant compositions of the present invention comprising: a reaction product of at least one heterocyclic compound chosen from lactone compounds and their thiocarbonyl

analogs, thiolactone compounds and their thiocarbonyl analogs, and lactam compounds and their thiocarbonyl analogs and at least one alkoxysilane having at least one amino substituent; and optionally, at least one carrier, are applied topically onto the desired area of a keratinous substrate in an amount sufficient to impart water resistance or hydrophobicity to the keratinous substrates.

In accordance with other preferred embodiments, the water resistant compositions of the present invention comprising at least one heterocyclic compound chosen from lactone compounds and their thiocarbonyl analogs, thiolactone compounds and their thiocarbonyl analogs, and lactam compounds and their thiocarbonyl analogs, at least one alkoxysilane having at least one amino substituent, and optionally, at least one carrier are applied topically to the desired area of the keratinous substrate in an amount sufficient to impart water resistance or hydrophobicity to the keratinous substrates.

In other embodiments of the present invention, the water resistant compositions of the present invention require at least one carrier.

In preferred embodiments, the disclosed compositions and reaction products of the present invention are applied onto substrates chosen from keratinous substrates such as skin and hair. In particular, the reaction product is combined with a benefit agent or with compositions containing benefit agents to improve the water resistance properties of compositions capable of providing at least one beneficial property to skin and hair, while at the same time, imparting durable or long lasting hydrophobicity as well as a protective barrier to skin and hair.

In certain embodiments, a method of providing a protective barrier onto a keratinous substrate is provided, wherein said method involves applying onto the keratinous substrate, a composition containing the above-described reaction product, and optionally, at least one carrier.

In one preferred embodiment of the present invention, there is provided, a composition for and a method of preventing or reducing ultraviolet light damage to a keratinous substrate, the method comprising applying onto the keratinous substrate, a composition containing the above-described reaction product; at least one sunscreen agent; and optionally, at least one carrier.

In other preferred embodiments of the present invention, there is provided, a method of imparting water resistance onto a substrate, involving applying onto the substrate, the compositions of the present invention.

The compositions of the present invention and the compositions containing benefit agents and capable of providing benefits to skin and hair may especially constitute cosmetic, personal care or dermatological compositions such as hair cosmetic compositions, hair care compositions, sunscreen compositions, makeup compositions, and skin care compositions.

In one embodiment, the composition of the present invention can be a hair cosmetic product such as a hair styling composition comprising (a) a reaction product of : (i) at least one heterocyclic compound chosen from $\gamma$-thiobutyrolactone, meadowfoam delta-lactone and mixtures therefore, and (ii) 3-aminopropyltriethoxysilane as described hereabove, a hair styling agent such as film formers and waxes, and a carrier.

In another embodiment, the composition of the present invention can be a hair cleansing composition comprising (a) a reaction product of : (i) at least one heterocyclic compound chosen from $\gamma$-thiobutyrolactone, meadowfoam delta-lactone and mixtures therefore, and (ii) 3-aminopropyltriethoxysilane as described hereabove, at least one surfactant chosen from anionic, nonionic, amphoteric/zwitterionic and cationic surfactants, and a carrier.

In yet another embodiment, the composition of the present invention can be a hair or a skin conditioning composition comprising (a) a reaction product of : (i) at least one heterocyclic compound chosen from $\gamma$-thiobutyrolactone, meadowfoam delta-lactone and mixtures therefore, and (ii) 3-aminopropyltriethoxysilane as described hereabove, a conditioning agent such as moisturizing agents, plant extracts, cationic and quaternary compounds, and a carrier.

In other embodiments, the composition of the present invention can be a makeup product comprising (a) a reaction product of : (i) at least one heterocyclic compound chosen from $\gamma$-thiobutyrolactone, meadowfoam delta-lactone and mixtures therefore, and (ii) 3-aminopropyltriethoxysilane as described hereabove, a first benefit agent comprising at least one colorant, optionally, a second benefit agent such as conditioning agents, moisturizing agents, emollients, sunscreen agents, and film forming agents, and a carrier.

In yet other embodiments, the composition of the present invention can be a skin care product comprising (a) a reaction product of : (i) at least one heterocyclic compound chosen from $\gamma$-thiobutyrolactone, meadowfoam delta-lactone and mixtures therefore, and (ii) 3-aminopropyltriethoxysilane as described hereabove, a benefit agent such as sunscreen agents, cosmetically active agents, dermatologically active agents, humectants, moisturizing agents, film forming agents, conditioning agents, emollients, vitamins, plant extracts, and pharmaceutical agents, and a carrier.

The composition of the present invention may also be used as a post treatment composition wherein it is applied onto hair and skin which has previously been contacted with a cosmetic, personal care or dermatological composition. An example of such a composition is a topcoat composition such as lip gloss or lip balm or a sun protectant composition.

The compositions of the present invention can be provided in a plethora of forms, including but not limited to creams, liquid, gel, cream-gel, lotion, foam, serum, paste, semi-solid, solid stick, stick-gel, or a powder, and may be in the form of a mousse or a spray, and may optionally be packaged as an aerosol, prepared according to the usual methods.

The compositions of the present invention may also be in the form of cleaning products and coatings which can be applied onto non-keratinous substrates such as glass, wood, metal, paper and fabric.

EXAMPLES

Water resistance evaluation

Water resistance of compositions for application onto skin was evaluated according to an in vitro water resistance test for skin ("WR Test I") that is based on a measurement of the quantity one or more organic sunscreens recovered from a substrate initially treated with a composition containing the organic sunscreen(s) after water immersion. The substrate consists of polymethylmethacrylate (PMMA)-coated plates ("PMMA plates") from Europlast, Inc. and were found to be suitable substitute substrates for skin (see Ahn, S., Yang, N., Lee, H. Alternative Evaluation Method In Vitro for the Water Resistant Effect of Sunscreen Products, J. of Skin Research and Technology. July 22, 2007.) The in vitro water resistance test for skin according to the present invention was employed as an alternative to in vivo testing on skin. The test is as follows:

A measured amount (18-20 mg) of a test composition (test sample) containing the organic sunscreen was distributed onto the surfaces of 5 PMMA plates to form treated plates and allowed to dry for 30 to 40 minutes in the dark. Two of the treated plates were designated as the control plates. The rest of the treated plates were designated as the test plates. The test plates were then attached to holders and immersed in a water bath for a specified period of time, such as 30 minutes, wherein the water was stirred at constant speed by a propeller device and the temperature of the water was at 23 degrees centigrade. The plates were then allowed to dry for 30 minutes. The composition remaining on the plates was twice extracted by 35 ml of alcohol (in this case, methanol) from the plate. The combined alcohol extract was diluted to 100 ml with alcohol to form a sample extract solution ("sample extract") from which a 9 ml aliquot was taken and diluted to 25 ml. The absorbance of the final extract solution for each sample was obtained by measuring the ultraviolet (UV) absorbance maximum.

The control plates were not subjected to the water immersion step described above. However, the same alcohol extraction procedure as above was performed on the control plates and the UV absorbance maximum for the control sample extract solution ("control extract") was measured.

The percent water-resistance (%WR) of the test composition is determined as:

% WR (WR Test I) = RA (sample extract)/RA(control extract) x 100

wherein RA is the relative absorbance such that:

RA (sample extract) = Absorbance (sample extract)/Weight of test composition; and

RA (control extract) = Absorbance (control extract)/Weight of test composition*

* weight of test composition on control plates.

When comparing % WR among several compositions, a higher % WR means that a higher amount of the composition remained on the plate, indicating that the composition is more water resistant.

Water Resistance Study on Hair

Water resistance of compositions for application onto hair was evaluated according to an in vitro water resistance test for hair ("WR Test II"). Various types of hair, including permed, bleached, relaxed or virgin hair may be used. The WR Test II is based on a measurement of the quantity a water soluble organic sunscreen (designated as a "marker") that is released/dissolves into water after immersing the hair in water, wherein the hair was initially treated with a composition containing the marker sunscreen. The concentration of organic sunscreen that is released into the water is directly proportional to the degree of water resistance or hydrophobicity imparted to the hair by the composition.

The treatment of the hair samples may performed in one of two ways: (A) directly treated with a test/control composition containing the marker sunscreen ("WR Test IIA"); or (B) first treated with an aqueous solution containing the marker sunscreen, then treated with the test/control composition ("WR Test IIB"). The hair samples may be treated by spraying, massaging, spreading or combing the test/control composition onto the hair or by dipping the hair into said composition. The treated hair samples for both control and test compositions are dried and placed in small perforated baskets. The baskets are then immersed in water and rotated at a constant speed for a period of time (e.g., 180 minutes) at 23 degrees centigrade. The concentrations of benzophenone-4 that is removed or released from the treated hair samples are obtained by measuring the UV absorbance maxima of the water based on a calibration curve.

The percent water-resistance (%WR) of the test composition is determined as:

% WR (WR Test IIA or IIB) = 100 - [RA(Test)/RA(Control) x 100]

wherein RA is the relative absorbance such that

RA (test) = Absorbance (Test)/Weight of treated hair before water immersion; and

RA (control) = Absorbance (Control)/Weight

of treated hair before water immersion.

When comparing % WR among several compositions, a higher % WR means that a higher amount of the composition remained on the plate, indicating that the composition is more water resistant.

Example I - Comparative Water-Resistance Study following WR Test I

The following skin care creams (test compositions) were formulated:

| Ingredients / % weight | A (base cream) | B | C | D | E | F |
|---|---|---|---|---|---|---|
| Emulsifier | 2.8 | 2.8 | 2.8 | 2.8 | 2.8 | 2.8 |
| Co-emulsifier | 4.0 | 4.0 | 4.0 | 4.0 | 4.0 | 4.0 |
| Structurant | 3.2 | 3.2 | 3.2 | 3.2 | 3.2 | 3.2 |
| Organic sunscreen(s) (ethylhexyl salicylate, octocrylene and butyl methoxydibenzoylmethane) | 15 | 15 | 15 | 15 | 15 | 15 |
| Humectant | 7 | 7 | 7 | 7 | 7 | 7 |
| Solvent | 7 | 7 | 7 | 7 | 7 | 7 |
| Preservatives | 1.6 | 1.6 | 1.6 | 1.6 | 1.6 | 1.6 |
| ε-Caprolactam | - | 4 | - | - | - | - |
| γ-Thiobutyrolactone | - | - | 4 | - | - | - |
| APTES | - | - | - | 4 | - | - |
| ε-Caprolactam/APTES* | - | - | - | - | 4 | - |
| γ - Thiobutyrolactone/APTES* | - | - | - | - | - | 4 |
| Water | 59.4 | 55.4 | 55.4 | 55.4 | 55.4 | 55.4 |

*The reaction product was prepared by heating a mixture of a 1:1 molar ratio of ε-Caprolactam or γ-Thiobutyrolactone and 3-aminopropyltriethoxysilane (APTES) under anhydrous conditions for 1 hour at 100°C.

Creams A, B, C, D, E, F were assessed for their water-resistance properties using the WR Test I described above.

The results are:

| Test Compositions | % WR |
|---|---|
| A (Base only) | 80 |
| B (Base + 4% by weight ε-Caprolactam) | 81 |
| C (Base + 4% by weight γ-Thiobutyrolactone) | 80 |
| D (Base + 4% APTES) | 81 |
| E (Base + 4% by weight ε-Caprolactam/APTES reaction product, 1:1 molar ratio) | 87 |
| F (Base + 4% by weight γ-Thiobutyrolactone/APTES reaction product, 1:1 molar ratio) | 93 |

The results from the water resistance test above show that the degree of water resistance by the inventive compositions, Creams E and F, is higher and therefore, significantly better than that of the other creams that did not contain both ε-Caprolactam and APTES or γ-Thiobutyrolactone and APTES since the higher the %WR, the more water-resistant the composition is. This result also demonstrates that the composition containing the reaction product provided water-resistant and long lasting and durable hydrophobicity onto a surface, which would extend the beneficial effects provided by the sunscreens or any active ingredient in the cream composition when such a composition is applied onto

keratinous substrates such as skin or hair.

Example 2 - Comparative Water-Resistance Study following WR Test I

The following skin care creams (test compositions) were formulated:

| Ingredients / % weight | A (base cream) | B | C | D |
|---|---|---|---|---|
| Emulsifier | 2.8 | 2.8 | 2.8 | 2.8 |
| Co-emulsifier | 4.0 | 4.0 | 4.0 | 4.0 |
| Structurant | 3.2 | 3.2 | 3.2 | 3.2 |
| Organic sunscreen(s) (ethylhexyl salicylate, octocrylene and butyl methoxydibenzoylmethane) | 15 | 15 | 15 | 15 |
| Humectant | 7 | 7 | 7 | 7 |
| Solvent | 7 | 7 | 7 | 7 |
| Preservatives | 1.8 | 1.8 | 1.8 | 1.8 |
| meadowfoam delta-lactone | - | 2 | - | - |
| APTES | - | - | 2 | - |
| meadowfoam delta-lactone /APTES (1:1 molar ratio*) | - | - | - | 2 |
| Water | ~~57~~59.2 | 57.2 | 57.2 | 57.2 |

*The reaction product was prepared by heating a mixture of a 1:1 molar ratio of meadowfoam delta-lactone and 3-aminopropyltriethoxysilane (APTES) under anhydrous conditions for 1 hour at 100°C.

Creams A, B, C, D were assessed for their water-resistance properties using the WR Test I described above.

The results are:

| Test Compositions | % WR |
|---|---|
| A (Base only) | 83 |
| B (Base + 2% by weight meadowfoam delta-lactone) | 81 |
| C (Base + 2% by weight APTES) | 82 |
| D (Base + 2% by weight meadowfoam delta-lactone /APTES reaction product, 1:1 molar ratio) | 94 |

The results from the water resistance test above show that the degree of water resistance by the inventive composition, Cream D, is higher and therefore, significantly better than that of the other creams that did not contain both meadowfoam delta-lactone and APTES since the higher the %WR, the more water-resistant the composition is. This result also demonstrates that the composition containing the reaction product provided water-resistant and long lasting and durable hydrophobicity onto a surface, which would extend the beneficial effects provided by the sunscreens or any active ingredient in the cream composition when such a composition is applied onto keratinous substrates such as skin or hair.

Example 3 - Water resistance Study on Hair following WR Test IIA

Control and test leave-on hair gel compositions containing 1% by weight of an organic sunscreen, benzophenone-4, were applied onto bleached hair samples (1 gram of gel per gram of hair) to form treated hair samples. The test hair gel composition contained 4% by weight of the reaction product according to the invention. The control hair gel composition did not contain the reaction product. Following the WR Test IIA described above, the treated hair samples were immersed in water for 180 minutes and the benzophenone-4 concentration in the water and %WR were measured.

The results are:

| Compositions applied onto the hair samples | Benzophenone-4 concentration (ppm) | %WR |
|---|---|---|
| Hair gel composition* (control) | 34.4 | |
| Hair gel composition containing 4% by weight meadowfoam delta-lactone/ APTES reaction product, 1:1 molar ratio** (test - inventive composition) | 22.4 | 37.83 |
| *The gel composition contained 3% cetearyl alcohol, 1% cremophore, 3% sclerotium gum, 1% benzophenone-4 and water (Q.S. to 100%) wherein the percentages are by weight based on the total weight of the composition.<br>*Each reaction product was prepared by heating a mixture of a 1:1 molar ratio of meadowfoam delta-lactone and APTES under anhydrous conditions for 30 minutes at 120°C. | | |

The results above show that the hair gel composition containing the reaction product was significantly more water resistant than the composition that did not contain the reaction product as evidenced by the lower amount of benzophenone-4 that was present in the water. This demonstrates that the hair gel composition containing the reaction product provided water resistance properties and long lasting and durable hydrophobicity onto the hair and at the same time, inhibited the water from permeating through or washing the composition off the hair.

Example 4 - Water Resistance Study on Hair following the WR Test IIB

Bleached hair samples were soaked in a 1% by weight of benzophenone-4 in water solution for 30 minutes and allowed to dry overnight at room temperature. Ethanol-based control and test compositions as tabulated below were then applied onto the hair samples for 30 minutes and the resulting treated hair samples were allowed to air dry. Following the WR Test IIB described above, the treated hair samples were immersed in water for 180 minutes and the benzophenone-4 concentration in the water and %WR were measured.

The results are:

| Compositions applied onto the hair samples | Benzophenone-4 concentration ($\mu$g/ml) |
|---|---|
| 1: ethanol (control) | 57.0 |
| 2: 1% meadowfoam delta-lactone in ethanol (test) | 55.7 |
| 3: 1% APTES in ethanol (test) | 70.9 |
| 4: 1% of the meadowfoam delta-lactone /APTES reaction product, 1:1 molar ratio*, in ethanol (test - inventive composition) | 40.4 |
| *Each reaction product was prepared by heating a mixture of a 1:1 molar ratio of meadowfoam delta-lactone and APTES under anhydrous conditions for 1 hour at 100°C. | |

The calculated %WR for composition 4 was 18.5%.

The results above show that composition 4, containing the reaction product, was significantly more water resistant as evidenced by the lower amount of benzophenone-4 that was present in the water compared to amounts of benzophenone-4 corresponding to the other compositions which did not contain the reaction product. This demonstrates that the composition containing the reaction product provided water resistance properties and long lasting and durable hydrophobicity onto the hair; at the same time, it inhibited the water from permeating through the hair and washing the composition off the hair.

## Claims

1. A composition comprising:

   (a) a reaction product of:

      (i) at least one heterocyclic compound chosen from γ-thiobutyrolactone, meadowfoam delta-lactone and mixtures thereof; and
      (ii) 3-aminopropyltriethoxysilane; and

   (b) optionally, at least one carrier; and
   (c) at least one benefit agent chosen from sunscreen agents, cosmetically active agents, dermatologically active agents, humectants, moisturizing agents, colorants, hair straightening/relaxing agents, film forming agents, shine agents, conditioning agents, reducing agents, emollients, vitamins, antidandruff agents, plant extracts, antiperspirants, and pharmaceutical agents;

   wherein the composition is water resistant.

2. The composition of claim 1, wherein (a)(i) is present in an amount of from 0.01 to 70 % by weight, preferably from 0.05 to 50% by weight, more preferably from 0.1 to 10% by weight, based on the total weight of the reaction product.

3. The composition of any preceding claim, wherein (a) (ii) is present in an amount of from 0.01 to 70 % by weight, preferably from 0.05 to 50% by weight, more preferably from 0.5 to 10% by weight, based on the total weight of the reaction product.

4. The composition of any preceding claim, wherein the molar ratio of (a) (i) to (a) (ii) ranges from 5:1 to 1:5, preferably from 3:1 to 1:3.

5. The composition of any preceding claim, wherein (a) is present in an amount of from 0.05 to 40% by weight, preferably from 0.1 to 30% by weight, more preferably from 0.5 to 10 % by weight, based on the total weight of the composition.

6. The composition of any preceding claim, wherein (b) comprises at least one compound chosen from water, oils, alcohols, organic solvents, esters, silicones, waxes, and mixtures thereof, and preferably (b) is a non-reactive solvent.

7. The composition of any preceding claim, wherein the composition is anhydrous.

8. A method of imparting water resistance onto a keratinous substrate, the method comprising applying onto the keratinous substrate, a composition comprising:

   (a) a reaction product of:

      (i) at least one heterocyclic compound chosen from lactone compounds and their thiocarbonyl analogs, thiolactone compounds and their thiocarbonyl analogs, and lactam compounds and their thiocarbonyl analogs; and
      (ii) at least one alkoxysilane having at least one amino substituent; and

   (b) optionally, at least one carrier.

## Patentansprüche

1. Zusammensetzung, umfassend:

   (a) ein Reaktionsprodukt von:

      (i) wenigstens einer heterocyclischen Verbindung ausgewählt aus γ-Thiobutyrolacton, Meadowfoam-deltalacton und Gemischen davon; und
      (ii) 3-Aminopropyltriethoxysilan; und

(b) gegebenenfalls wenigstens einen Träger; und
(c) wenigstens ein nutzbringendes Mittel ausgewählt aus Sonnenschutzmitteln, kosmetischen Wirkstoffen, dermatologischen Wirkstoffen, Feuchthaltemitteln, Befeuchtungsmitteln, Farbstoffen, haarglättenden/entspannenden Mitteln, filmbildenden Mitteln, Glanzmitteln, Konditionierungsmitteln, Reduktionsmitteln, Weichmachern, Vitaminen, Antischuppenmitteln, Pflanzenextrakten, Antiperspirants und pharmazeutischen Mitteln;

wobei die Zusammensetzung wasserbeständig ist.

**2.** Zusammensetzung gemäß Anspruch 1, wobei (a) (i) in einer Menge von 0,01 bis 70 Gew.-% enthalten ist, vorzugsweise von 0,05 bis 50 Ges.-%, bevorzugter von 0,1 bis 10 Gew.-%, bezogen auf das Gesamtgewicht des Reaktionsprodukts.

**3.** Zusammensetzung gemäß einem der vorstehenden Ansprüche, wobei (a) (ii) in einer Menge von 0,01 bis 70 Gew.-% enthalten ist, vorzugsweise von 0,05 bis 50 Gew.-%, bevorzugter von 0,5 bis 10 Gew.-%, bezogen auf das Gesamtgewicht des Reaktionsprodukts.

**4.** Zusammensetzung gemäß einem der vorstehenden Ansprüche, wobei das Molverhältnis von (a) (i) zu (a)(ii) in dem Bereich von 5:1 bis 1:5 liegt, vorzugsweise von 3:1 bis 1:3.

**5.** Zusammensetzung gemäß einem der vorstehenden Ansprüche, wobei (a) in einer Menge von 0,05 bis 40 Gew.-% enthalten ist, vorzugsweise von 0,1 bis 30 Gew.-%, bevorzugter von 0,5 bis 10 Gew.-%, bezogen auf das Gesamtgewicht der Zusammensetzung.

**6.** Zusammensetzung gemäß einem der vorstehenden Ansprüche, wobei (b) wenigstens eine Verbindung ausgewählt aus Wasser, Ölen, Alkoholen, organischen Lösungsmitteln, Estern, Siliconen, Wachsen und Gemischen davon umfasst und vorzugsweise (b) ein nichtreaktives Lösungsmittel ist.

**7.** Zusammensetzung gemäß einem der vorstehenden Ansprüche, wobei die Zusammensetzung wasserfrei ist.

**8.** Verfahren, einem keratinischen Substrat Wasserbeständigkeit zu verleihen, wobei das Verfahren Aufbringen einer Zusammensetzung umfassend

(a) ein Reaktionsprodukt von:

(i) wenigstens einer heterocyclischen Verbindung ausgewählt aus Lactonverbindungen und ihren Thiocarbonylanaloga, Thiolactonverbindungen und ihren Thiocarbonylanaloga und Lactamverbindungen und ihren Thiocarbonylanaloga; und
(ii) wenigstens einem Alkoxysilan mit wenigstens einem Aminosubstituenten; und

(b) gegebenenfalls wenigstens einen Träger,

auf das keratinische Substrat umfasst.

## Revendications

**1.** Composition comprenant :

(a) un produit de réaction

(i) d'au moins un composé hétérocyclique choisi parmi la gamma-thiobutyrolactone, la delta-lactone de limnanthe, et leurs mélanges, et
(ii) de 3-amino-propyl-triéthoxy-silane ; et

(b) optionnellement, au moins un véhicule ; et
(c) au moins un agent bénéfique choisi parmi les agents écrans solaires, les agents cosmétologiquement actifs, les agents dermatologiquement actifs, les agents humectants, les agents humidifiants, les colorants, les agents de défrisage/relaxation des cheveux, les agents filmogènes, les agents de brillance, les agents de conditionnement, les agents réducteurs, les émollients, les vitamines, les agents antipelliculaires, les extraits végétaux, les agents anti-transpiration et les agents pharmaceutiques ;

ladite composition étant résistante à l'eau.

2. Composition selon la revendication 1, **caractérisée en ce que** (a)-(i) est présent en une proportion allant de 0,01 à 70 % en poids, de préférence de 0,05 à 50 % en poids et mieux encore de 0,1 à 10 % en poids, par rapport au poids total du produit de réaction.

3. Composition selon l'une quelconque des revendications précédentes, **caractérisée en ce que** (a)-(ii) est présent en une proportion allant de 0,01 à 70 % en poids, de préférence de 0,05 à 50 % en poids et mieux encore de 0,5 à 10 % en poids, par rapport au poids total du produit de réaction.

4. Composition selon l'une quelconque des revendications précédentes, **caractérisée en ce que** le rapport molaire de (a)-(i) à (a)-(ii) vaut de 5/1 à 1/5, et de préférence de 3/1 à 1/3.

5. Composition selon l'une quelconque des revendications précédentes, **caractérisée en ce que** (a) est présent en une proportion allant de 0,05 à 40 % en poids, de préférence de 0,1 à 30 % en poids et mieux encore de 0,5 à 10 % en poids, par rapport au poids total de la composition.

6. Composition selon l'une quelconque des revendications précédentes, **caractérisée en ce que** (b) comprend au moins un composé choisi parmi l'eau, les huiles, les alcools, les solvants organiques, les esters, les silicones, les cires, et leurs mélanges, (b) étant de préférence un solvant non-réactif.

7. Composition selon l'une quelconque des revendications précédentes, **caractérisée en ce qu'**elle est anhydre.

8. Procédé permettant de conférer de la résistance à l'eau sur un substrat kératinique, lequel procédé comporte le fait d'appliquer sur le substrat kératinique une composition comprenant :

(a) un produit de réaction

(i) d'au moins un composé hétérocyclique choisi parmi les lactones et leurs analogues thiocarbonylés, les thiolactones et leurs analogues thiocarbonylés, et les lactames et leurs analogues thiocarbonylés, et
(ii) d'au moins un alcoxy-silane portant au moins un substituant amino ; et

(b) optionnellement, au moins un véhicule.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 2789896
- EP 1216023 A
- WO 9206778 A


### Non-patent literature cited in the description


- **AHN, S. ; YANG, N. ; LEE, H.** Alternative Evaluation Method In Vitro for the Water Resistant Effect of Sunscreen Products. *J. of Skin Research and Technology,* 22 July 2007